## Europäisches Patentamt

European Patent Office

Office européen des brevets

(11) Publication number: **0 165 006**
**B1**

(12) **EUROPEAN PATENT SPECIFICATION**

(45) Date of publication of patent specification: **12.12.90**

(51) Int. Cl.⁵: **C 07 D 251/24, C 07 D 251/22**

(21) Application number: **85303955.0**

(22) Date of filing: **04.06.85**

(54) Processes for preparing substituted halomethyl-s-triazines.

(30) Priority: **04.06.84 US 617042**

(43) Date of publication of application:
**18.12.85 Bulletin 85/51**

(45) Publication of the grant of the patent:
**12.12.90 Bulletin 90/50**

(84) Designated Contracting States:
**DE FR GB**

(56) References cited:
**US-A-3 954 475**

(73) Proprietor: **Polychrome Corporation**
**137 Alexander Street**
**Yonkers New York 10702 (US)**

(72) Inventor: **Rowe, William**
**RR. 1, PO Box 103**
**Califon New Jersey 07830 (US)**
Inventor: **Dooley, Thomas**
**975 Teaneck Road**
**Teaneck New Jersey 07666 (US)**
Inventor: **Shah, Ajay**
**241 West Grand Street Apt. C-5**
**Elizabeth New Jersey 07202 (US)**

(74) Representative: **Lawrence, Peter Robin**
**Broughton et al**
**GILL JENNINGS & EVERY 53-64 Chancery Lane**
**London WC2A 1HN (GB)**

The file contains technical information
submitted after the application was filed and
not included in this specification

Courier Press, Leamington Spa, England.

## Description

There is a need to make 2-(p methoxy styryl)-4,6 bis (trichloro-methyl)-s-triazine, referred to below as the "end compound". Two step methods of making this compound are described in U.S. Patents 3,987,037 and 3,954,475.

In particular, U.S. 3,954,475 shows that the end compound may be made by reacting 2,4-bis (trichloromethyl)-6-methyl-s-triazine, which is referred to hereinafter as the "intermediate compound" under conditions typical of the well known Knoevenagel reaction for the condensation of aldehydes with reactive methylene compounds. In Example 1 the reaction is conducted in toluene and a yield of 77% is quoted, but the size of the reaction mixture and the basis of the yield are not disclosed.

It is stated in U.S. 3,954,475 that the said intermediate compound can be made by reaction of organic nitriles and haloacetonitriles in accordance with the teachings of Wakabayashi et al., Bulletin of the Chemical Society of Japan, 42, 2924—30 (1969). This describes the reaction of trichlorocacetonitrile with acetonitrile (or other nitriles) in the presence of hydrogen halide or, preferably, a Friedel-Crafts catalyst-hydrogen halide complex. Various combinations of Friedel-Crafts catalyst and hydrogen halide are shown to be suitable. In particular, a process is shown for the production of the said intermediate compound by reaction of 289 g trichloroacetonitrile and 50 g acetonitrile with Friedel-Crafts catalyst and hydrogen chloride followed by melting and pouring into water, cooling, separating and drying. In another process, the product is made using hydrogen chloride alone and the product is heated to 150—200°C and fractionated undec reduced pressure.

When making the intermediate product for use in the Knoevenagel reaction on a large scale for commerical operations isolation of this intermediate compound has proved to be quite difficult, since the crude reaction product must be melted by heating to 110°C and then subsequently added to water to isolate very sticky material. Consequently a large excess of water and much time is required in order to isolate and purify the intermediate compound.

It would be desirable to have available a process that could be readily adaptable for commercial and continuous operations which avoided the disadvantages of the known two step procedures based on the said article by Wakabayashi and U.S. 3,954,475. It would be particularly advantageous to have a process which did not require the time consuming and cumbersome step of separating the intermediate compound from the crude reaction product obtained in the initial step of the overall procedure, and yet which gave a satisfactory yield, especially when scaled up to an industrial plant size.

A process according to the invention for preparing the end compound comprises reacting acetonitrile and trichloroacetonitrile in the presence of a Friedel-Crafts catalyst and hydrogen chloride to form a crude reaction product comprising the intermediate compound and reacting the said intermediate compound with anisaldehyde in the presence of a condensation catalyst to form the end compound, and the process is characterised in that the said crude reaction product is dissolved in toluene, an inert gas is passed through the toluene solution to remove excess hydrogen chloride, water is added to the solution, insoluble Friedel-Crafts catalyst residue is separated from the solution and the resultant solution is mixed with the anisaldehyde and the condensation catalyst.

Thus the two-step processes of the prior art are modified by forming the solution of the crude reaction product containing the intermediate compound, treating this solution in the defined manner, and then using the resultant solution to introduce the intermediate compound into the Knoevenagel reaction.

The yield of the end product may be increased by running the Knoevenagel reaction with an initial charge of the condensation catalyst and, when evolution of water ceases, separating the product by filtration and adding a second charge of condensation catalyst to the filtrate and repeating the reaction.

In the invention, the initial step involves reacting acetonitrile with trichloroacetonitrile in the presence of a powdered, anhydrous Freidel-Crafts catalyst such as aluminium bromide or aluminium chloride. The mixture is preferably cooled, e.g., to minus 5°C, and that temperature is maintained throughout the reaction. Hydrogen chloride should then be added, generally by bubbling gas into the mixture over a prolonged period, for instance 4 to 7 hours, preferably to saturate the mixture.

The resulting product is a thick, white slurry and after standing for a while the slurry will turn into a white solid mass.

Solvent that is preferably part or all of the solvent that is to be used in the second part of the reaction is then added. The preferred solvent is toluene but others that may be used include pyridine, benzene, ethyl acetate, methanol and ethanol. This solution of the solid in the solvent may be promoted by heating, for instance to 70 to 100°C for a period of 2 to 3 hours.

Substantially all of the hydrogen chloride should be removed from the solution by purging the solution with an insert gas, generally nitrogen or argon or mixtures thereof, for as long as is necessary.

The purged toluene solution may then be cooled to room temperature and admixed with water. The solution may then be filtered to remove insolubles, which are belived to be decomposition products of the Friedel-Crafts catalyst and possibly polymeric byproducts.

The organic layer is then separated from the aqueous layer and is dried, for instance over sodium sulphate, so as to produce a substantially anhydrous solution of the methyl halomethyl-s-triazine.

In the second step the condensation reaction of methyl halomethyl-s-triazine with aldehydes takes place under conditions typical of the known Knoevenagel reaction. Particular details are described in U.S. Patent 3,987,037, especially in the paragraph bridging columns 4 and 5.

The reaction is conveniently effected by mixing the described intermediate solution with a suspension of the chosen condensation catalyst in a suitable organic solvent, generally toluene. After stirring the catalyst and intermediate solutions the p-anisaldehyde may be added and the mixture may then be heated to promote reaction. Preferably reaction is caused by heating to reflux under a nitrogen sparge. The reaction temperature will generally range from about 100 to 110°C and heating will be continued for a time period of at least 5 hours, and preferably from 5 to 7 hours. Preferably the reaction is continued until the evolution of water ceases. The water evolved from the heated admixture may be recovered and separated.

The resulting reaction product is than cooled to room temperature and product filtered off.

The filtrate may be admixed with a second portion of a toluene suspension of the catalyst, e.g., piperidine acetate. After stirring the resulting mixture the reaction product mixture may again be heated to reflux and water collected overhead. The reaction may be continued for another 5 to 7 hours or at least until no water is collected overhead.

The final reaction product mixture may be cooled to room temperature, filtered, and washed with petroleum ether and then with ethanol. The solid material is the desired product, i.e., Triazine D. If desired, it can be further purified by being recrystallised from a mixture of ethyl acetate and ethanol.

The following is an Example of the invention.

Example

In a 12 litre reaction flask, kept in an ice-salt bath, and equipped with a stirrer, condenser, thermometer, gas inlet and outlet tubes, gas traps, etc., were mixed 3719 g pre-cooled trichloroacetonitrile and 644 g of pre-cooled acetonitrile 45 g of powdered anhydrous aluminium bromide with stirring. The temperature was brough down to −5°C, and the mixture was maintained at that temperature throughout the reaction.

Hydrogen chloride gas was bubbled into the mixture for about 5 hours until saturation was attained and a thick white slurry developed. Approximately 3 lbs of the hydrogen chloride gas was utilised. After the resulting reaction product mixture was allowed to reach room temperture gradually, standing overnight, the reaction product mixture become a solid, white mass.

Approximately 4 kilograms of toluene was added to the solid, white reaction product. The resulting mixture is gradually heated to 80°C over a 3 hour period. At that temperature the toluene solution was purged with nitrogen gas under agitation to remove the hydrogen chloride gas from the system. The toluene solution was cooled to room temperature, admixed with 500 ml of deionized water under stirring for 4 hours. Insolubles were filtered from the toluene solution followed by separation of the water layer therefrom. The toluene layer was dried over anhydrous sodium sulphate.

The toluene solution is admixed with a catalyst comprising a toluene suspension of piperidine acetate. The catalyst suspension was prepared in a 22 litre reaction flask by adding thereto 1500 g toluene and 314 g piperidine at 25°C with agitation and over a 30 minute period portions of 222 g glacial acetic acid. Strong exotherms up to 50°C were observed as a white suspension formed. This suspension was stirred for at least 5 hours while protecting it from moisture, and left overnight or it may be prepared a few days prior to condensation of the toluene reaction mixture with p-anisaldehyde.

To the admixture of the catalyst and the toluene reaction mixture solution, 1780 g of p-anisaldehyde were added with stirring. The resulting mixture was heated to reflux under a nitrogen sparge. The reaction was carried out for 7 hours at the reflux temperature of about 110°C. Water is collected overhead in two Dean's traps. The reaction may be stopped once water evolution ceases. The mixture is then cooled to room temperature, and the precipitated crude product is filtered off.

A second catalyst addition (the preparation of which is described above) was next added to the filtrate and then the temperature was raised to the reflux temperature of 110°C. Again, the reaction was carried out for 7 hours or until water evolution ceased. A total of approximately 210 ml of water was collected.

The reaction mixture was then cooled and filtered to recover the solid product, which was crude Triazine D. The Triazine D was purified by being washed twice with petroleum ether and once with cold ethanol. The weight of the product was 3300—3500 g (crude yield equalled about 63—68%).

If further purification of the product is desired, it can be carried out by using a solvent mixture of ethyl acetate: ethyl alcohol in a ratio of solvent mixture to crude of 7.5:1 (w/w) and with the ethyl acetate to ethyl alcohol ratio of 2.4:1.

The purified, yellow material weighed about 3000 g which was a yield of 58% (based on trichloroacetonitrile). The melting point was 190—192°C, extinction value (the intensity of absorption times 0.1 concentration in grams per litre) was 77.5 at 377 nm in acetone.

## Claims

1. A process for preparing the end compound 2-(p-methoxy styryl)-4,6-bis (trichloromethyl)-s-triazine comprising reacting acetonitrile and trichloroacetonitrile in the presence of the Friedel-Crafts catalyst and hydrogen chloride to form a crude reaction product comprising the intermediate compound 2,4-bis (trichloromethyl)-6-methyl-s-triazine and reacting the said intermediate compound with anisaldehyde in the presence of a condensation catalyst to form the end compound, characterised in that the said crude reaction product is dissolved in toluene, an inert gas is passed through the solution to remove excess hydrogen chloride, water is added to the solution, insoluble Friedel-Crafts catalyst residue is separated from the solution and the resultant solution is mixed with the anisaldehyde and the condensation catalyst.

2. A process according to claim 1 in which water is separated from the solution after separating the insoluble components and the solution is then dried.

3. A process according to either preceding claim in which the organic solvent is toluene.

4. A process according to any preceding claim in which the Friedel-Crafts catalyst is selected from aluminium bromide and aluminium chloride.

5. A process according to any preceding claim in which the inert gas is nitrogen.

6. A process according to any preceding claim in which the yield of the vinyl-halomethyl-s-triazine is increased by running the reaction with an initial charge of condensation catalyst and when evolution of water ceases separating the product by filtration, and then adding a second charge of condensation catalyst to the filtrate and repeating the reaction.

7. A process according to any preceding claim in which the condensation catalyst is piperidine acetate.

**Patentansprüche**

1. Verfahren zur Herstellung der Endverbindung 2-(p-Methoxystyryl)-4,6-bis(trichlormethyl)-s-triazin, welches die Reaktion von Acetonitril und Trichloracetonitril in Gegenwart des Friedel-Crafts Katalysators und Salzsäure zur Bildung eines Reaktionsrohprodukts, das die Zwischenverbindung 2,4-Bis(trichlormethyl)-6-methyl-s-triazin umfaßt, und die Reaktion der Zwischenverbindung mit Anisaldehyd in Gegenwart eines Kondenstationskatalysators zur Bildung der Endverbindung umfaßt, dadurch gekennzeichnet, daß man das Reaktionsrohprodukt in Toluol löst, ein inertes Gas durch die Lösung leitet, um überschüssige Salzsäure zu entfernen, Wasser zur Lösung zugibt, den unlöslichen Rückstand des Friedel-Crafts Katalysators von der Lösung abtrennt und die resultierende Lösung mit Anisaldehyd und dem Kondensationskatalysator mischt.

2. Verfahren nach Anspruch 1, worin Wasser von der Lösung abgetrennt wird, nachdem die unlöslichen Bestandteile abgetrennt wurden, und die Lösung dann getrocknet wird.

3. Verfahren nach einem der vorhergehenden Ansprüche, worin das organische Lösungsmittel Toluol ist.

4. Verfahren nach einem der vorhergehenden Ansprüche, worin der Freidel-Crafts Katalysator aus Aluminiumbromid und Aluminiumchlorid ausgewählt ist.

5. Verfahren nach einem der vorangehenden Ansprüche, worin das Inertgas Stickstoff ist.

6. Verfahren nach einem der vorhergehenden Ansprüche, worin die Ausbeute an Vinyl-halogenmethyl-s-triazin erhöht wird durch Ausführen der Reaktion mit einer Anfangscharge an Kondensationskatalysator und, wenn die Wasserbildung aufgehört hat, Abtrennen des Produkts durch Filtration, und anschließende Zugabe einer zweiten Charge des Kondensationskatalysators zum Filtrat und Wiederholung der Reaktion.

7. Verfahren nach einem der vorhergehenden Ansprüche, worin der Kondensationskatalysator Piperidinacetat ist.

**Revendications**

1. Un procédé pour fabriquer le composé final 2-(p-méthoxy-styryl)-4,6-bis(trichlorométhyl)-s-triazine consistant à faire réagir l'acétonitrile et le trichloroacétonitrile en présence du catalyseur de Friedel-Crafts et de chlorure d'hydrogène pour former un produit brut de réaction comprenant le composé intermédiaire 2,4-bis(trichlorométhyl)-6-méthyl-s-triazine et à faire réagir ledit composé intermédiaire avec l'anisaldéhyde en présence d'un catalyseur de condensation pour former le composé final, caractérisé en ce que ledit produit brut de réaction est dissous dans le toluène, on fait passer un gaz inerte à travers la solution pour séparer l'excès de chlorure d'hydrogène, on ajoute de l'eau à la solution, on sépare de la solution le résidu insoluble de catalyseur de Friedel-Crafts et on mélange la solution résultante avec l'anisaldéhyde et le catalyseur de condensation.

2. Un procédé selon la revendication 1, dans lequel l'eau est séparée de la solution après séparation des composants insolubles et la solution est ensuite séchée.

3. Un procédé selon l'une quelconque des revendications précédentes, dans lequel le solvant organique est le toluène.

4. Un procédé selon l'une quelconque des revendications précédentes, dans lequel le catalyseur de Friedel-Crafts est choisi parmi le bromure d'aluminium et le chlorure d'aluminium.

5. Un procédé selon l'une quelconque des revendications précédentes, dans lequel le gaz inerte est l'azote.

6. Un procédé selon l'une quelconque des revendications précédentes, dans lequel le rendement en vinyl-halogénométhyl-s-triazine est augmenté en effectuant la réaction avec une charge initiale du catalyseur de condensation et, lorsque le dégagement d'eau cesse, en séparant le produit par filtration et en ajoutant ensuite au filtrat une seconde charge de catalyseur de condensation et en répétant la réaction.

7. Un procédé selon l'une quelconque des revendications précédentes, dans lequel le catalyseur de condensation est l'acétate de pipéridine.